# EUROPÄISCHE PATENTSCHRIFT

(11) **EP 2 215 980 B1**
(45) Veröffentlichungstag und Bekanntmachung des Hinweises auf die Patenterteilung: **19.12.2012**
(21) Anmeldenummer: 09001517.3
(22) Anmeldetag: 04.02.2009
(51) Int. Cl.: A61B 17/16, A61B 17/00, A61C 1/00, H01H 9/04, A61C 17/00

(54) **Chirurgisches Elektrowerkzeug und Betätigungsbaugruppe hierfür**
Surgical electric tool and actuation components for same
Outil électrique chirurgical et composant d'actionnement correspondant

(43) Veröffentlichungstag der Anmeldung: 11.08.2010
(73) Patentinhaber: Stryker Leibinger GmbH & Co. KG, 79111 Freiburg (DE)
(72) Erfinder: Ippisch, Andreas, 79291 Merdingen (DE)
(74) Vertreter: Röthinger, Rainer

(56) Entgegenhaltungen:
- EP-A- 1 754 447
- DE-A1-102004 041 871
- US-A- 5 098 430
- US-A- 5 162 775

## Beschreibung

### Technisches Gebiet

Die Erfindung betrifft allgemein chirurgische Elektrowerkzeuge wie elektromotorisch betriebene Bohrer, Knochensägen und Schraubendreher. Genauer gesagt betrifft die Erfindung ein chirurgisches Elektrowerkzeug, das eine Betätigungsbaugruppe mit einem Kraftsensor umfasst.

### Technischer Hintergrund

Bereits seit mehreren Jahrzehnten verwenden Chirurgen bei ihren Tätigkeiten verschiedenste Elektrowerkzeuge. Herkömmliche chirurgische Elektrowerkzeuge umfassen dabei häufig mechanische Betätigungsbaugruppen mit Schiebeschaltern, Kippschaltern oder Drehknöpfen zur Steuerung bestimmter Werkzeugfunktionalitäten. Mechanische Betätigungsbaugruppen sind jedoch für chirurgische Elektrowerkzeuge, jedenfalls sofern diese sterilisiert werden müssen, mitunter nachteilig. Dies hängt damit zusammen, dass sich die beweglichen Komponenten solcher Baugruppen schlecht gegenüber dem Eintritt von flüssigen oder gasförmigen Sterilisationsmedien abdichten lassen.

Das Eindringen eines Sterilisationsmediums in mechanische Betätigungsbaugruppen ist deren Funktionsfähigkeit abträglich. Daher lassen sich chirurgische Elektrowerkzeuge mit Kippschaltern, Drehknöpfen oder ähnlichen beweglicher Komponenten entweder überhaupt nicht sterilisieren oder müssen nach wenigen Sterilisationszyklen gewartet werden.

Um die Sterilisierbarkeit chirurgischer Elektrowerkzeuge zu verbessern oder überhaupt erst zu ermöglichen, können Betätigungsbaugruppen mit einem Kraftsensor ausgerüstet werden. Kraftsensoren weisen eine planare Bauform auf und besitzen keine beweglichen mechanischen Elemente. Aus diesen Gründen lassen sich Kraftsensoren auf einfache und dichte Weise unterhalb eines flexiblen Gehäuseabschnitts eines chirurgischen Elektrowerkzeugs einbauen.

Chirurgische Elektrowerkzeuge mit unter flexiblen Gehäuseabschnitten angeordneten Kraftsensoren sind beispielsweise aus der US 3,463,990 oder der US 6,037,724 bekannt. Bei den in diesen Schriften beschriebenen Elektrowerkzeugen ist der jeweilige Kraftsensor innerhalb einer Kunststoffummantelung aufgenommen, die den Kraftsensor gegenüber Sterilisationsmedien schützt.

Aus der EP 1 754 447 A2 ist ferner ein chirurgisches Elektrowerkzeuge mit einem in einer metallischen Kapsel angeordneten Kraftsensor bekannt, gemäβ dem Obergriff des Ansprunchs 1. Die Kapselung schützt den Sensor zuverlässig vor Sterilisationsmedien. Zur Gewährleistung der Funktionsfähigkeit des gekapselten Kraftsensors wird eine fortlaufenden Kalibrierung vorgeschlagen.

Weitere Bedienelemente für chirurgische Werkzeuge werden zum Beispiel in den Dokumenten DE 10 2004 041 871 A1 und US 5,098,430 beschrieben. Dabei sind jeweils Taster durch elastische Kunststoffe vor den Sterilisationsmedien geschützt.

Der Erfindung liegt die Aufgabe zugrunde, die Funktionsfähigkeit bekannter chirurgischer Elektrowerkzeuge mit Kraftsensoren zu erhöhen.

### Kurzer Abriss

Gemäß einem ersten Aspekt wird eine Betätigungsbaugruppe zur betätigungskraftabhängigen Steuerung des Betriebs eines chirurgischen Elektrowerkzeugs vorgeschlagen, wobei die Betätigungsbaugruppe einen zur Erfassung der Betätigungskraft ausgebildeten Kraftsensor, ein Trägerbauteil für den Kraftsensor, das dem Kraftsensor in einer Krafteinleitungsrichtung vorgelagert und in Kraft übertragender Weise mit diesem gekoppelt ist, und ein mechanisches Dämpfungsglied, das dem Trägerbauteil in der Krafteinleitungsrichtung vorgelagert ist, umfasst.

Das mechanische Dämpfungsglied wirkt einer Beschädigung der Betätigungsbaugruppe durch Schläge oder Stöße im chirurgischen Umfeld entgegen. Zu diesem Zweck kann das Dämpfungsglied elastische oder federnde Eigenschaften aufweisen.

Was den Aufbau des Kraftsensors anbelangt, kann zwischen verschiedenen Realisieren ausgewählt werden. So ist es denkbar, den Kraftsensor als Dehnungsmessstreifen, als Piezoelement, als Halbleiterelement, usw. auszubilden. Für den Kraftsensor kann eine mit dem Kraftsensor elektrisch gekoppelte Signalaufbereitungsschaltung vorgesehen sein. Gemäß einer ersten Variante greift die Signalaufbereitungsschaltung ein Sensorsignal ab und wandelt dieses in ein betätigungskraftabhängiges, kontinuierliches Ausgangssignal. Gemäß einer zweiten Variante wandelt die Signalaufbereitungsschaltung das Sensorsignal in ein diskretes, also z. B. binäres (Ein/Aus) oder mehrstufiges, Ausgangssignal.

Der Kraftsensor kann vollständig oder teilweise gekapselt sein. Die Sensorkapsel kann zur Anordnung in, auf oder unter dem Gehäuse des chirurgischen Elektrowerkzeugs vorgesehen sein. Die Kapsel kann ganz oder teilweise aus einem gegenüber Sterilisationsmedien beständigen Material bestehen (oder mit einem solchen Material beschichtet sein). So kann die Kapsel ganz oder teilweise aus einem Metall gefertigt sein. Die Kapsel kann einen Überzug aus Kunststoff besitzen oder einen Kern aus einem nicht-metallischen Werkstoff, der mit einer Metallbeschichtung überzogen ist. Bei dem Trägerbauteil kann es sich um einen Teil der den Kraftsensor aufnehmenden Sensorkapsel handeln.

Gemäß einer Variante umfasst die Betätigungsbaugruppe ferner eine vorzugsweise flexible Abdeckung, welche über dem Trägerbauteil angeordnet ist. Das Dämpfungsglied kann in diesem Fall entweder in die Abdeckung integriert sein oder zwischen der Abdeckung und dem Trägerbauteil angeordnet sein. Auch ist es denkbar, diese beiden Varianten zu kombinieren, indem zwei oder mehr Dämpfungsglieder in Kraftübertragungsrichtung hintereinander angeordnet werden.

Die Abdeckung kann eine im Wesentlichen planare Gestalt aufweisen und sich im Wesentlichen senkrecht zur Krafteinleitungsrichtung erstrecken. Zusätzlich oder alternativ hierzu kann die Abdeckung auch im Wesentlichen parallel zum Trägerbauteil verlaufen.

Das Dämpfungsglied kann eine stellenweise, insbesondere konvexe Dickenerhöhung aufweisen oder von einer stellenweisen, insbesondere konvexe Dickenerhöhung der Abdeckung gebildet sein. Die stellenweise Dickenerhöhung gestattet es, einen für den Chirurgen haptisch gut erfassbaren Einleitungsbereich für die Betätigungskraft zu definieren. Der Chirurg kann so den Einleitungsbereich erfühlen, ohne notwendigerweise den Blick auf die Betätigungsbaugruppe bzw. das chirurgische Elektrowerkzeug richten zu müssen.

Gemäß einer Ausgestaltung besitzt das Dämpfungsglied eine minimale Dicke von ungefähr 1,5 mm und insbesondere von ungefähr 2 bis 2,5 mm. Während mit steigender Dicke die Dämpfungseigenschaften zunehmen, kann es gleichzeitig zu einer Beeinträchtigung der kraftübertragenden Eigenschaften kommen. Trotz der dämpfenden Eigenschaften kann das Dämpfungsglied starr genug ausgebildet sein, um bei einer Anordnung im Kraftübertragungsweg zwischen einem Krafteinleitungsbereich und dem Kraftsensor als Kraftübertragungsglied fungieren zu können. Je nach Ausgestaltung des Dämpfungsglieds und der Materialwahl kann sich die Dicke des Dämpfungsglieds in einem Bereich zwischen ungefähr 1,5 mm bis 5 mm, insbesondere zwischen 2 mm und 4 mm bewegen.

Sowohl das Dämpfungsglied als auch die optionale Abdeckung können aus einem gegenüber Sterilisationsmedien undurchlässigem Material bestehen. Elastische Polymermaterialien wie Silikon oder andere Kunststoffe können hier Verwendung finden.

Die Betätigungsbaugruppe kann als eine eigenständig handhabbare Unterbaugruppe des Elektrowerkzeugs ausgebildet sein. In diesem Fall kann die Betätigungsbaugruppe als Ganzes (jedenfalls mit ihren maßgeblichsten Komponenten) in ein Gehäuse des Elektrowerkzeugs eingefügt werden, was die Montage vereinfacht.

Die Betätigungsbaugruppe kann ferner eine Trägerplatte zur Aufnahme der Sensorkapsel umfassen. Die Trägerplatte kann dazu ausgebildet sein, eine in einem Gehäuse des Elektrowerkzeugs ausgebildete Öffnung zur Aufnahme der Betätigungsbaugruppe zu verschließen. Das Verschließen der Betätigungsbaugruppe durch die Trägerplatte kann in einer fluiddichten Weise erfolgen, um dem Eintritt eines Steriiisationsmediums in das Gehäuseinnere entgegen zu wirken. Zu diesem Zweck kann eine Dichtung zwischen der Trägerplatte und dem die Öffnung begrenzenden Gehäuseabschnitt vorgesehen sein.

Zur Fixierung und/oder Zentrierung der Sensorkapsel auf der Trägerplatte kann ein die Sensorkapsel zumindest teilweise umschließendes Lagerbauteil verwendet werden. Zu diesem Zweck kann das Lagerbauteil eine Aufnahmeöffnung für die Sensorkapsel besitzen. Die Trägerplatte und das Lagerbauteil können einstückig oder als getrennte Komponenten gefertigt sein.

Gemäß einem weiteren Aspekt wird ein chirurgisches Elektrowerkzeug vorgeschlagen, welches ein Gehäuse sowie ein im Bereich des Gehäuses angeordnete Betätigungsbaugruppe wie hier beschrieben umfasst. Das chirurgische Elektrowerkzeug kann ferner einen Elektromotor zur Betätigung eines Werkzeugelements (z.B. einer Schraubendreherklinge, eines Sägeblatts, eines Bohrers usw.) umfassen. Außerdem ist es denkbar, dass das chirurgische Elektrowerkzeug mehr als eine Betätigungsbaugruppe besitzt.

Das Gehäuse des Elektrowerkzeugs kann eine Öffnung zur Krafteinleitung in Richtung auf den Kraftsensor besitzen. Die Abdeckung kann die Öffnung gegenüber dem Eintritt von Sterilisationsmedien dicht verschließen. Zu diesem Zweck kann die Abdeckung im Rahmen der Montage mit einer die Abdeckung komprimierenden Kraft beaufschlagt werden, welche im fertig montiertem Zustand aufrechterhalten bleibt. Ferner ist es möglich, dass die Abdeckung eine die Öffnung des Gehäuses umgebende erste Oberflächenprofilierung aufweist, welche im Wesentlichen formschlüssig mit einer zweiten Oberflächenprofilierung des Gehäuses zusammenwirkt. Der hierbei entstehende Formschluss kann eine Barriere gegenüber dem Eintritt von Sterilisationsmedien bilden.

Ferner kann die Abdeckung dichtend zwischen einer ins Gehäuseinnere weisenden ersten Oberfläche des Gehäuses und einer der ersten Oberfläche zugewandten zweiten Oberfläche der Betätigungsbaugruppe angeordnet sein. Bei der ersten Oberfläche kann es sich um einen Hinterschnitt oder eine Auskragung des Gehäuses handeln. Die zweite Oberfläche kann an einem Lagerbauteil für das Trägerbauteil ausgebildet sein.

### Kurze Beschreibung der Zeichnungen

Weitere Aspekte und Vorteile der Erfindung ergeben sich aus der nachfolgenden Beschreibung bevorzugter Ausführungsbeispiele sowie aus den Figuren. Es zeigen:
- Fig. 1: eine Aufsicht eines Ausführungsbeispiels eines chirurgischen Elektro- werkzeugs in Gestalt eines Schraubendrehers;
- Fig. 2: eine Teilschnittansicht des chirurgischen Elektrowerkzeugs gemäß Fig. 1 entlang der Linie A-A;
- Fig. 3: eine Ausschnittsvergrößerung des Details Z der Schnittansicht gemäß Fig. 2, die insbesondere ein Ausführungsbeispiel einer zwei Betäti- gungsbaugruppen umfassenden Betätigungseinheit veranschaulicht;
- Fig. 4: eine schematische Teilschnittansicht einer Sensorkapsel einer der Betä- tigungsbaugruppen gemäß Fig. 3;
- Fig. 5: eine Schnittansicht eines Gehäuses der Sensorkapsel gemäß Fig. 4;
- Fig. 6: eine Aufsicht auf einen bei jeder der Betätigungsbaugruppen gemäß Fig. 3 zum Einsatz kommenden Kraftsensor in Gestalt eines Dehnungs- messstreifens;
- Fig. 7: eine Ausschnittsvergrößerung des Details Y der Schnittansicht gemäß Fig. 3, die insbesondere die Funktionsweise eines Schalters veranschau- licht;
- Fig. 8: eine perspektivische Ansicht eines bei dem Schalter gemäß Fig. 7 ver- wendeten Federblechs;
- Fig. 9: eine Seitenansicht des Federblechs gemäß Fig. 8; und
- Fign. 10A/B: ein schematisches Flussdiagramm, welches eine Ausführungsbeispiel eines Verfahrens zum Betreiben des chirurgischen Elektrowerkzeugs veranschaulicht.

### Detaillierte Beschreibung

Im Folgenden werden Ausführungsbeispiele eines chirurgischen Elektrowerkzeugs, einer hierfür vorgesehenen Betätigungsbaugruppe sowie eines hierfür geeigneten Betriebsverfahrens erläutert. Übereinstimmende Elemente sind mit übereinstimmenden Bezugszeichen versehen.

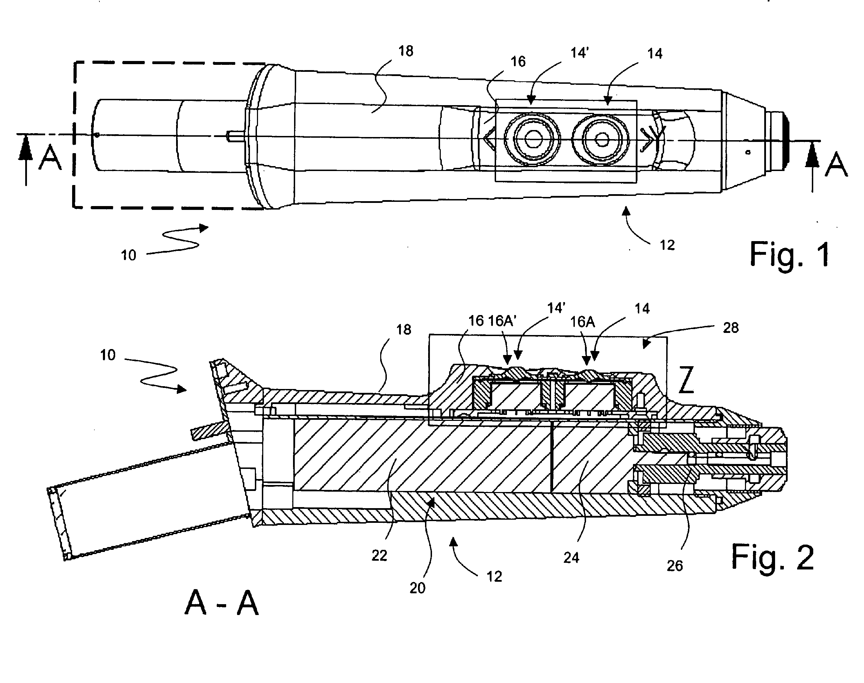

Fig. 1 zeigt eine Aufsicht eines chirurgischen Elektrowerkzeuges 10 in Gestalt eines batteriebetriebenen Schraubendrehers. Das chirurgische Elektrowerkzeug 10 besitzt ein lang gestrecktes, annähernd zylindrisches Gehäuse 12 aus Aluminium, an dessen Rückseite ein Batteriepack (nur schematisch und gestrichelt dargestellt) abnehmbar aufgesteckt werden kann.

Das chirurgische Elektrowerkzeug 10 umfasst im Ausführungsbeispiel zwei Betätigungsbaugruppen 14, 14' zur Steuerung verschiedener Werkzeugfunktionen. Die Betätigungsbaugruppen 14, 14' sind in einem dem Batteriepack abgewandten vorderen Bereich des Gehäuses 12 vorgesehen. Wie insbesondere der in Fig. 2 dargestellten Schnittansicht entlang der Linie A-A von Fig. 1 entnommen werden kann, sind die Betätigungsbaugruppen 14, 14' in einem Kragen 16 des Gehäuses 12 aufgenommen, der über einen zylindrischen Mantelbereich 18 des Gehäuses 12 übersteht. Der einstückig mit dem Mantelbereich 18 gefertigte Kragen 16 umgibt die Betätigungsbaugruppen 14, 14' seitlich und schützt diese vor mechanischen Einwirkungen. Auf seiner Oberseite weist der Kragen 16 zwei kreisrunde Öffnungen 16A, 16A' auf, um einem Benutzer einen Zugriff auf die Betätigungsbaugruppen 14, 14' zu ermöglichen.

Wie in Fig. 2 dargestellt, ist im Inneren des Gehäuses 12 eine Baugruppe 20 mit einem elektronisch kommutierter Motor 22 und einem mit dem Motor 22 gekoppelten Getriebe 24 vorgesehen. Eine erste der beiden Betätigungsbaugruppen 14, 14' steuert den Elektromotor 22 in einer ersten Drehrichtung. Die andere der Betätigungsbaugruppen 14, 14' steuert den Elektromotor 22 in einer zur ersten Drehrichtung entgegengesetzten zweiten Drehrichtung. Die Motordrehzahl in Vorwärts- und Rückwärtsrichtung wird jeweils proportional zur Betätigungskraft, die in die jeweilige Betätigungsbaugruppe 14, 14' eingeleitet wird, geregelt. Je höher die Betätigungskraft ist, desto höher ist also die Motordrehzahl. Zur Drehzahlregelung ist eine Motoransteuerschaltung auf einer im rückwärtigen Teil des Gehäuses 12 befestigten Platine (nicht dargestellt) vorgesehen.

Außerdem ist im Gehäuse 12 getriebeabwärts eine Kupplung 26 aufgenommen. Die Kupplung 26 gestattet in bekannter Weise die drehfeste Kopplung einer austauschbaren Schraubendreherklinge (nicht dargestellt) mit dem Getriebe 24. Ein optionaler Sperrknopf (ebenfalls nicht dargestellt) ermöglicht eine drehfeste mechanische Arretierung der Kupplung 24. Ist der Sperrknopf betätigt, lässt sich das Elektrowerkzeug 10 wie ein herkömmlicher Schraubendreher verwenden. Das Drehmoment wird dann also nicht von dem Motor 22 erzeugt, sondern durch manuelle Drehung des Gehäuses 12 aufgebracht.

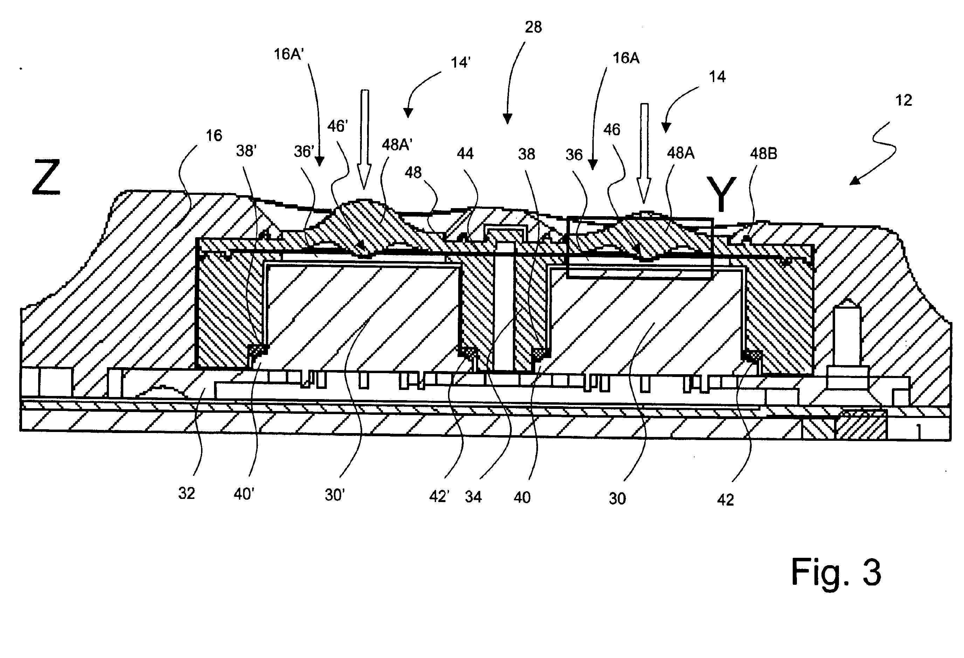

Die insgesamt zwei Betätigungsbaugruppen 14, 14' des chirurgischen Elektrowerkzeugs besitzen den gleichen Aufbau und bilden zusammen eine eigenständig handhabbare und als Unerbaugruppe in das Gehäuse 12 einsetzbare Betätigungseinheit 28. Fig. 3 zeigt die Betätigungseinheit 28 in einer Ausschnittsvergrößerung des Details Z der Schnittansicht gemäß Fig. 2.

Wie Fig. 3 zu entnehmen ist, umfassen die Betätigungsbaugruppen 14, 14' jeweils eine Sensorkapsel 30, 30' aus Metall zur hermetischen Verkapselung jeweils eines Kraftsensors (in Fig. 3 nicht dargestellt). Die Sensorkapseln 30, 30' sind auf einer gemeinsamen Trägerplatte 32 angeordnet, welche eine auf der Unterseite des Kragens 16 ausgebildete Gehäuseöffnung verschließt. Auf der Trägerplatte 32 sind die Sensorkapseln 30, 30' mittels eines gemeinsamen, die Sensorkapseln 30, 30' zentrierenden Lagerbauteils 34 fixiert. Zu diesem Zweck weist das blockförmig ausgebildete Lagerbauteil 34 zwei zylindrische Bohrungen 36, 36' zur Aufnahme jeweils einer Sensorkapsel 30, 30' auf. Das Lagerbauteil 34 besteht aus einem isolierenden Material wie Kunststoff oder ist anderweitig gegenüber den metallischen Sensorkapseln 30, 30' elektrisch isoliert.

Die Bohrungen 36, 36' im Lagerbauteil 34 sind als Durchgangsöffnungen ausgebildet und gestatten einen Zugang zu elektrischen Kontakten der Sensorkapseln 30, 30' von unten und das Einleiten einer Betätigungskraft auf die Sensorkapseln 30, 30' von oben. Ferner weisen die Bohrungen 36, 36' ein gestuftes Profil mit jeweils einer umlaufenden Schulter 38, 38' auf, die als Anlage für eine Durchmessererweiterung 40, 40' jeder Sensorkapsel 30, 30' fungiert. Zwischen jeder Schulter 38, 38' und Durchmessererweiterung 40, 40' ist eine Dichtung 42, 42' in Gestalt eines Silikonringes vorgesehen. Die Dichtungen 42, 42' verhindern den Eintritt eines Sterilisationsmediums entlang der Seitenwände der Sensorkapseln 30, 30' und der Innenwände der Bohrungen 36, 36' in Richtung auf die Trägerplatte 32 und das Innere des Werkzeuggehäuses 12. Außerdem zentrieren die Dichtungen 42, 42' die Sensorkapseln 30, 30' bei deren Montage im Lagerbauteil 34. Zu diesem Zweck können die Dichtungen 42, 42' eine geeignete Profilierung aufweisen (z.B. eine in Richtung auf die Achse der Bohrungen 36, 36' abnehmende Dicke).

Auf der Oberseite des Lagerbauteils 34 sind ein planares Federblech 44 sowie eine elastische Abdeckung 48 aus Silikon oder einem anderen geeigneten Material vorgesehen. Die Abdeckung 48 ist dichtend zwischen einem Hinterschnitt des Kragens 16 und einer Oberseite des Lagerbauteils 34 angeordnet und verhindert so den Eintritt von Sterilisationsmedien durch die Gehäuseöffnungen 16A, 16A' in das Innere des Kragens 16 und in das Werkzeuggehäuse 12.

Um die Abdichtwirkung zu optimieren, besitzt die Abdeckung 48 eine Mehrzahl von konzentrisch zu den Gehäuseöffnungen 16A, 16A' angeordneten Oberflächenprofilierungen (in Fig. 3 ist der Klarheit halber lediglich eine einzige Oberflächenprofilierung 48B eingezeichnet). Die Oberflächenprofilierungen der Abdeckung 48 sind als kreisrunde Vorsprünge ausgebildet und wirken formschlüssig mit zugeordneten Oberflächenprofilierungen des Kragens 16 und des Lagerbauteils 34 in Gestalt von korrespondierenden Vertiefungen zusammen. Der auf diese Weise entstehende Formschluss wirkt dem Eintritt von Sterilisationsmedien in einer Richtung parallel zur Abdeckung 48 entgegen.

Um die Sensorkapseln 30, 30' (und die darin aufgenommenen Kraftsensoren) vor Schlägen und Stößen im chirurgischen Umfeld zu schützen, besitzt jede der beiden Betätigungsbaugruppen 14, 14' je ein mechanisches Dämpfungsglied 48A, 48A', welches den Sensorkapseln 30, 30' in Krafteinleitungsrichtung vorgelagert ist. Die Krafteinleitungsrichtung ist in Fig. 3 für jede der Betätigungsbaugruppen 14, 14' durch einen Blockpfeil veranschaulicht.

Die mechanischen Dämpfungsglieder 48A, 48A' sind in dem in Fig. 3 dargestellten Ausführungsbeispiel in die Abdeckung 48 integriert. Genauer gesagt sind die Dämpfungsglieder 48A, 48A' als konvexe Dickenerhöhungen der Abdeckung 48 oberhalb des zentralen Bereichs jeder der beiden Sensorkapseln 30, 30' ausgebildet. Aufgrund der konvexen Formgebung definieren die Dämpfungsglieder 48A, 48A' einen für den Benutzer haptisch gut erfassbaren Einleitungsbereich für die Betätigungskraft. In dem Ausführungsbeispiel gemäß Fig. 3 besitzen die Dämpfungsglieder 48A, 48A' eine Materialstärke von ungefähr 2,5 bis 3,5 mm in ihrem dicksten Bereich.

Wie Fig. 3 zu entnehmen ist, ist das Federblech 44 zwischen der Abdeckung 48 und den Oberseiten der Sensorkapseln 30, 30' angeordnet. In Bezug auf jede der beiden Betätigungsbaugruppen 14, 14' ist das Federblech 44 Teil je eines Schalter 46, 46'. Genauer gesagt stellt das Federblech 44 für jeden Schalter 46, 46' jeweils einen ersten Kontakt bereit. Der jeweils zweite Schaltkontakt wird von den (metallischen) Oberseiten der Sensorkapseln 30, 30' bereitgestellt. In dem in Fig. 3 dargestellten ersten Schaltzustand der Schalter 46, 46' sind die beiden jeweiligen Schaltkontakte durch die Federkraft des Federblechs 44 voneinander beabstandet gehalten. Die beiden Schalter 46, 46' befinden sich also in einem geöffneten Schaltzustand.

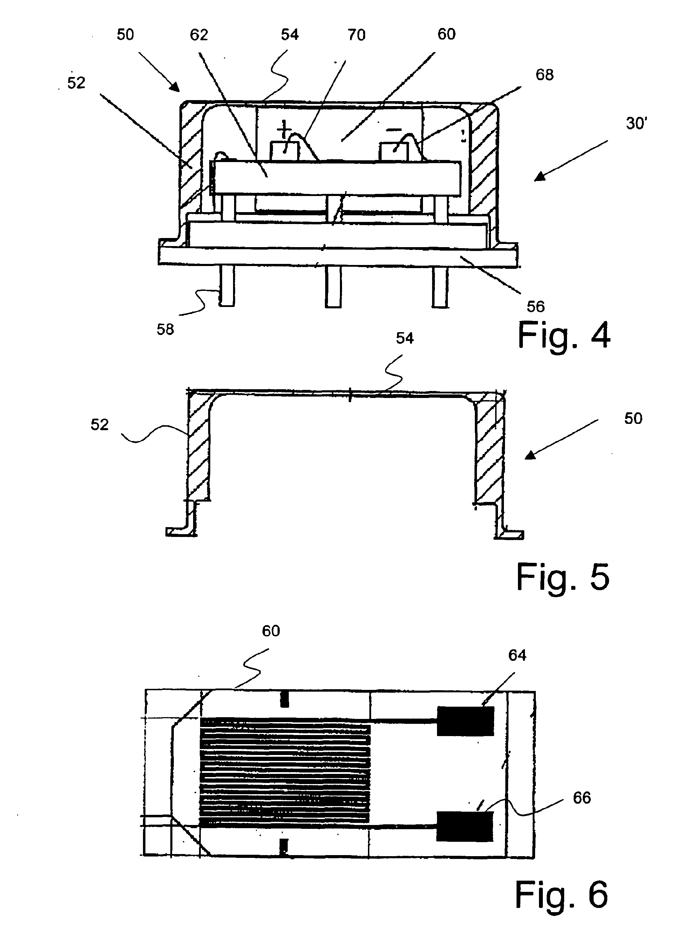

Im Folgenden wird der Aufbau der Sensorkapseln 30, 30' sowie der Schalter 46, 46' unter Bezugnahme auf die Fign. 4 bis 9 näher erläutert. Fig. 4 zeigt eine Teilschnittansicht der Sensorkapsel 30 der Betätigungsbaugruppe 14. Die Kapsel 30 besitzt eine im Wesentlichen topfförmige Kappe 50 aus Edelstahl, die in Fig. 5 nochmals einzeln dargestellt ist. Die Kappe 50 umfasst einen zylindrischen Mantelabschnitt 52 sowie einen einstückig mit dem Mantelabschnitt 52 ausgebildeten Deckelabschnitt 54. Der Innendurchmesser des Mantelabschnitts 52 beträgt ungefähr 11 mm (typischerweise ca. 5 bis 30 mm) und die Höhe des Mantelabschnitts 52 ungefähr 7 mm (typischerweise ca. 2 bis 12 mm). Der Deckelabschnitt 54 verschließt die in den Fig. 4 obere Stirnseite des Mantelabschnitts 52. Die offene untere Stirnseite der Kappe 50 ist durch einen Kappenboden 56 hermetisch gegenüber Sterilisationsmedien verschlossen. Der Kappenboden 56 besteht ebenfalls aus Edelstahl.

Im Kappenboden 56 sind mehrere Durchgangsöffnungen (nicht dargestellt) ausgebildet. Durch jede Durchgangsöffnung erstreckt sich je ein vergoldeter elektrischer Kontakt 58. Um einerseits die Kontakte 58 zu stabilisieren und um andererseits eine hohe Dichtheit zu gewährleisten, sind die Öffnungen im Kappenboden 56 mittels Glas hermetisch verschlossen.

Während der Deckelabschnitt 54 eine Stärke von ungefähr maximal 0,3 mm aufweist, besitzt der Mantelabschnitt 52 eine Stärke von wenigstens ungefähr 0,8 mm oder darüber (vgl. Fig. 5). Eine solche Ausführung ist vorteilhaft, um bei Einleiten einer Betätigungskraft in den Deckelabschnitt 54 die resultierende elastische Verformung auf den Deckelabschnitt 54 zu beschränken. Mit anderen Worten verhält sich der Mantelabschnitt 52 im Wesentlichen starr bezüglich der in den Deckelabschnitt 54 eingeleiteten Betätigungskraft. Dies erleichtert den hermetisch dichten Einbau der Sensorkapsel 30 in die Betätigungsbaugruppe 14 und in das Gehäuse 12 des Elektrowerkzeugs 10.

Innerhalb der Kapsel 50 sind ein Kraftsensor 60 sowie eine Signalaufbereitungsschaltung 62 für den Kraftsensor 60 aufgenommen. Fig. 6 zeigt eine Aufsicht auf den Kraftsensor 60. Der Kraftsensor 60 umfasst einen in Mäander-Form ausgebildeten planaren Dehnungsmessstreifen mit zwei Kontakten 64, 66. Im fertig montierten Zustand sind die Kontakte 64, 66 elektrisch mit der Signalaufbereitungsschaltung 62 verbunden. Die Montage des Kraftsensors 60 flach auf der Innenseite des Deckelabschnitts 54 kann durch Verkleben erfolgen.

Wie in Fig. 4 gezeigt, ist der Kraftsensor 60 mittels elektrischer Kontaktierungen 68, 70 mit der Signalaufbereitungsschaltung 62 gekoppelt. Die Signalaufbereitungsschaltung 62 wiederum wird von den aus der Kapsel 30 heraus führenden Kontakten 58 elektrisch kontaktiert.

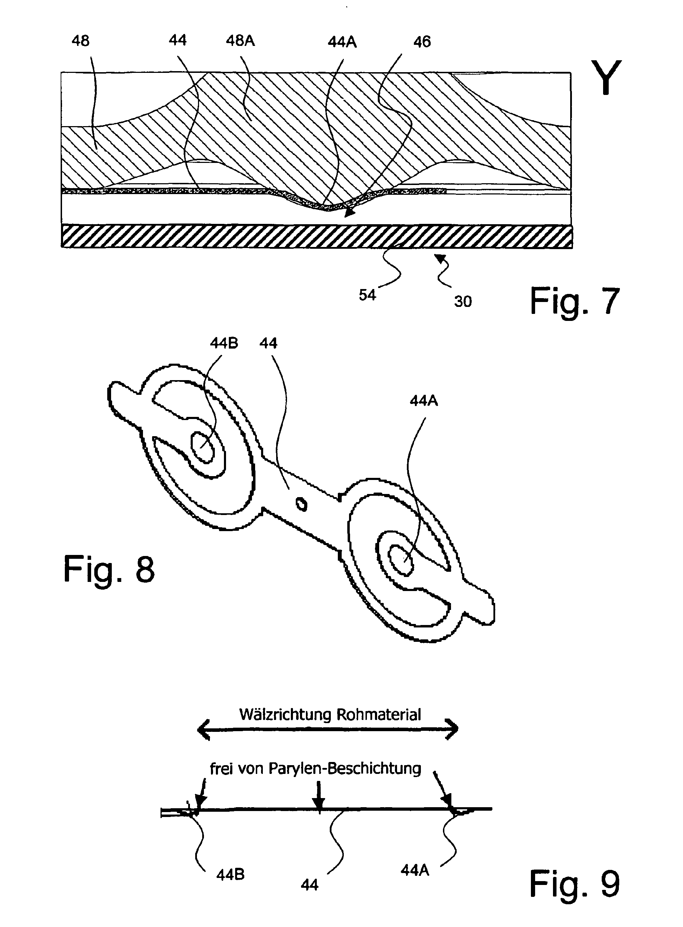

Nun wird unter Bezugnahme auf die Fign. 7 bis 9 der Aufbau der Schalter 46, 46' beschrieben. In diesem Zusammenhang wird zuerst auf Fig. 7 und die dort dargestellte Ausschnittsvergrößerung des Details Y in Fig. 3 Bezug genommen. In Fig. 7 deutlich zu erkennen ist der Aufbau des Schalters 46 aus einem ersten, am Federblech 44 ausgebildeten Schaltkontakt 44A und einem zweiten Schaltkontakt, der von dem metallischen Deckelabschnitt 54 der Sensorkappe 50 gebildet wird.

Wie der in Fig. 8 veranschaulichten Formgebung des Federblechs 44 entnommen werden kann, wird der Schaltkontakt 44A des Schalters 46 von einem zungenförmigen Federblechabschnitt gebildet, der an einer Stelle mit einem kreisringförmigen weiteren Federblechabschnitt verbunden ist. Der kreisringförmige Federblechabschnitt liegt auf dem in Fig. 3 dargestellten Lagerbauteil 34 auf, während der zungenförmige Federblechbereich (mit dem Schaltkontakt 44A) senkrecht zur Federblechebene gegen eine Federkraft auslenkbar ist. Diese Auslenkung erfolgt elastisch reversibel, so dass das Federblech 44 im Anschluss an eine Auslenkung wieder seine ursprüngliche planare Gestalt annimmt.

Fig. 9 zeigt eine Seitenansicht des Federblechs 44. In Fig. 9 ist darüber hinaus durch einen Pfeil die Wälzrichtung bei der Herstellung des Federblechs 44 angedeutet. Das Federblech 44 ist beidseits mit einem elektrisch isolierenden Material wie beispielsweise Parylen beschichtet. Lediglich drei Kontaktstellen sind von dieser Beschichtung ausgenommen, nämlich der Schaltkontakt 44A, ein entsprechender Schaltkontakt 44B der zweiten Betätigungsbaugruppe 14' sowie ein in der Mitte des Federblechs 44 ausgebildeter Kontaktbereich, über den ein Stromkreis geschlossen wird. Wie in den Fign. 7 und 9 dargestellt, besitzt der Schaltkontakt 44A eine konkav gewölbte Gestalt, um einen definierten, punktförmigen Kontaktschluss zwischen dem Schaltkontakt 44A und dem in Fig. 7 gegenüberliegenden Schaltkontakt in Gestalt des Deckelabschnitts 54 herzustellen.

Nun wird die Funktionsweise der Betätigungsbaugruppe 14 unter Bezugnahme auf die Fign. 3, 4 und 7 näher erläutert. Es versteht sich, dass die folgenden Ausführungen auch auf die Funktionsweise der zweiten Betätigungsbaugruppe 14' zutreffen.

Bei Einleiten einer Betätigungskraft (beispielsweise durch Fingerdruck) in den gut erfühlbaren verdickten Abschnitt 48A der elastischen Abdeckung 48 wird diese in Richtung auf das Gehäuseinnere verschoben. Von dieser Verschiebung der Abdeckung 48 wird auch der in Fig. 7 dargestellte Schaltkontakt 44A erfasst, der im Ausgangszustand an der Abdeckung 48 anliegt. Genauer gesagt wird der Schaltkontakt 44A gegen die von dem Federblech 44 bereit gestellte Federkraft in Richtung auf den Deckelabschnitt 54 verschoben. Nach einem Verschiebeweg von ungefähr 0,5 mm (typischerweise ca. 0,1 bis 2 mm) gelangt der Schaltkontakt 44A in Anlage an den Deckelabschnitt 54. In Folge dieses in Anlage gelangens wird der Schalter 46 geschlossen, d.h. er wird von einem offenen Zustand in einen geschlossenen Zustand überführt. Das Schließen des Schalters 46 bewirkt gleichzeitig das mittels einer Logikschaltung detektierbare Schließen eines Stromkreises, welcher das Federblech 44 und die Sensorkapsel 30 als leitende Elemente umfasst. Bei einer alternativen, nicht dargestellten Ausführungsform ist der Schalter 46 ausgebildet, um durch die Betätigungskraft geöffnet zu werden.

Sobald der Schaltkontakt 44A in Anlage an den Kappendeckel 54 gelangt ist, bewirkt eine weitere Erhöhung der Betätigungskraft das Einleiten eines Betätigungskraftanteils in den Deckelabschnitt 54. Die Oberseite des Deckelabschnitts 54 gestattet das Aufnehmen dieses Betätigungskraftanteils. Der Deckelabschnitt 54 verformt sich daraufhin elastisch in Richtung auf das Innere der Kapsel 30. Diese Verformung des Deckelabschnitts 54 überträgt sich auf den Kraftsensor 60, der auf der Unterseite des Deckelabschnitt 54 befestigt ist (vgl. Fig. 4). Genauer gesagt bewirkt die Verformung eine Dehnung des als Dehnungsmessstreifen ausgebildeten Kraftsensors 60. Infolge dieser Dehnung ändert sich der Widerstand des Kraftsensors 60. Diese Widerstandsänderung des Kraftsensors 60 wiederum verschiebt den Betriebspunkt einer Brückenschaltung, welche den Kraftsensor 60 umfasst und zusammen mit einer Verstärkerschaltung die Signalaufbereitungsschaltung 62 bildet. Der Kraftsensor 60 ist Teil der Brückenschaltung, die neben drei weiteren Brückenwiderständen auch noch zwei Abgleichswiderstände umfasst. Eine geeignete Schaltung ist beispielsweise aus der EP 1 754 447 A2 bekannt.

Die Verschiebung des Betriebspunktes wird von der als Differenzverstärker ausgebildeten Verstärkerschaltung der Signalaufbereitungsschaltung 62 erfasst und in ein verstärktes Differenzsignal umgesetzt. Das verstärkte Differenzsignal wird von der Signalaufbereitungsschaltung 62 als Ausgangssignal zur Weiterverarbeitung bereitgestellt. Der Pegel des Ausgangssignals ist proportional zur Verformung des Dehnungsmessstreifens und damit auch proportional zu der in den Deckelabschnitt 54 eingeleiteten Betätigungskraft. Bei einer alternativen Ausführungsform ist die Signalaufbereitungsschaltung derart ausgebildet, dass das Ausgangssignal zwei oder mehr diskrete Pegel (z. B. in Abhängigkeit von der Überschreitung einer oder mehrerer Kraftschwellen) besitzt.

Eine Motoransteuerungsschaltung ist elektrisch mit den Signalaufbereitungsschaltungen der Betätigungsbaugruppen 14, 14' gekoppelt. Funktionell zwischen der Motoransteuerungsschaltung und den beiden Betätigungsbaugruppen 14, 14' ist die eine Logikschaltung angeordnet. Die Logikschaltung bewirkt im Wesentlichen, dass sich bei gleichzeitiger Krafteinleitung in die beiden Betätigungsbaugruppen 14, 14' kein undefinierter Zustand einstellt. Zu diesem Zweck besitzt die Logikschaltung zwei getrennte Eingangsanschlüsse, die jeweils mit einer der beiden Betätigungsbaugruppen 14, 14' gekoppelt sind. Sofern nur an einem einzigen der beiden Eingangsanschlüsse ein Signal anliegt, wird über genau einen von zwei Ausgangsanschlüssen ein verstärktes Ausgangssignal an die Motoransteuerschaltung weitergeleitet. Über einen ersten Ansteueranschluss wird der Motoransteuerschaltung ein Signal für die erste Drehrichtung und über einen zweiten Ansteueranschluss ein Signal für die entgegengesetzte zweite Drehrichtung zugeführt.

Liegen an beiden Eingangsanschlüssen der Logikschaltung Ausgangssignale an (d.h. wird in beide Betätigungsbaugruppen 14, 14' eine Betätigungskraft eingeleitet), bewirkt die in der Logikschaltung implementierte Logik, dass an keinem der beiden Ausgangsanschlüsse ein Ausgangssignal an die Motoransteuerungsschaltung ausgegeben wird. Außerdem nimmt ein "Brake"-Anschluss einen hohen Signalpegel an. Der hohe Signalpegel am "Brake"-Anschluss schließt den elektronisch kommutierten Elektromotor 22 kurz, wodurch der Elektromotor 22 elektrisch abgebremst wird und zum Stillstand gelangt. Die Logikschaltung umfasst ferner einen Geschwindigkeitsregel-Ausgang. Über den Geschwindigkeitsregel-Ausgang erhält die Motoransteuerungsschaltung eine Rückmeldung über die angeforderte Motordrehzahl. Eine geeignete Logikschaltung ist beispielsweise aus der EP 1 754 447 A2 bekannt. Die bekannte Logikschaltung kann noch um Logik-Elemente ergänzt werden, welche die Ausgangssignale der Schalter 46, 46' sowie der vorstehend erläuterten Ausgangsanschlüsse verknüpft (z.B. mittels einer UND-Verknüpfung), um die nachfolgend erläuterte Plausibilisierung zu implementieren.

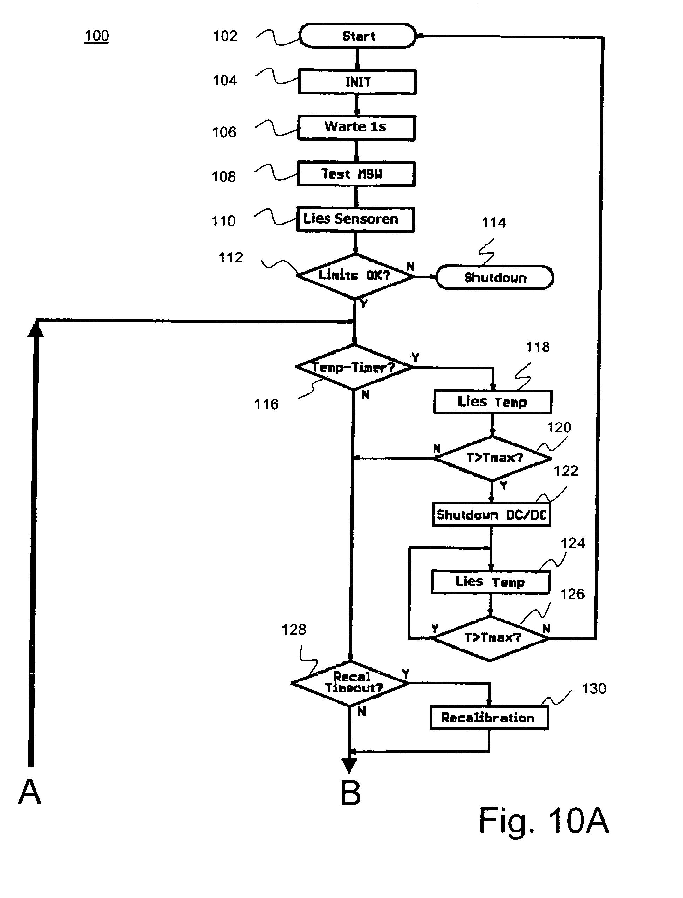

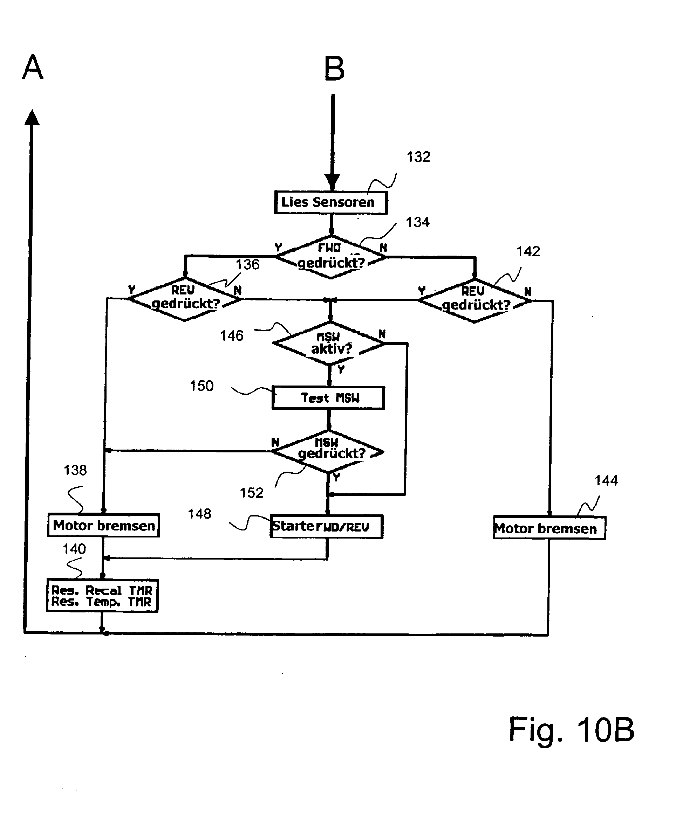

Im Folgenden wird anhand des schematischen Flussdiagramms 100 gemäß den Fign. 10A und 10B der Betrieb des chirurgischen Elektrowerkzeugs 10 ausführlicher beschrieben. Das Betriebsverfahren beginnt in Schritt 102 mit dem Aufstecken eines Batteriepacks auf den in Fig. 1 dargestellten Werkzeuggrundkörper und die damit einhergehende Initialisierung der einzelnen Werkzeugschaltungen in Schritt 104. Im Anschluss an den Initialisierungsschritt 104 und ein Warteintervall (Schritt 106) erfolgt in Schritt 108 ein Überprüfung der Funktionsfähigkeit der beiden Schalter 46, 46' (im Folgenden auch Hauptschalter oder "MSW" genannt). Hierbei wird insbesondere überprüft, ob beide Schalter 46, 46' sich in ihrem in Fig. 3 dargestellten, offenen Schaltzustand befinden. Sollte sich einer der beiden Schalter 46, 46' bereits im Initialisierungszustand des Elektrowerkzeugs 10 in einem geschlossenen Schaltzustand befinden, deutet dies auf eine Fehlfunktion (beispielsweise auf ein verbogenes Federblech 44 oder einen Fluideintritt) hin.

Gleichzeitig oder zeitlich getrennt von dem Testen der Schalter 46, 46' erfolgt in Schritt 110 ein Auslesen der Kraftsensorsignale der Betätigungsbaugruppen 14, 14'. In einem sich daran anschließenden Überprüfungsschritt 112 wird ermittelt, ob die Ausgangssignale der Kraftsensoren innerhalb eines vorgegebenen Bereichs (z.B. oberhalb vorgegebener Untergrenzen und unterhalb vorgegebener Obergrenzen) liegen. Ein Unterschreiten einer Untergrenze oder Überschreiten einer Obergrenze im Initialisierungszustand deutet auf eine Fehlfunktion (beispielsweise auf eine plastische Deformation eines Deckelabschnitts 54) hin. Sollte in Schritt 112 festgestellt werden, dass für wenigstens einen der Kraftsensoren die Untergrenze unterschritten oder die Obergrenze überschritten ist oder dass sich einer der Schalter 46, 46' in einem geschlossenen Zustand befindet, wird zu Schritt 114 verzweigt und ein Betrieb des Elektrowerkzeugs 10 gesperrt. Gleichzeitig kann ein akustisches Signal ausgegeben werden, welches auf die Fehlfunktion hinweist.

Wenn hingegen in Schritt 112 keine Fehlfunktion festgestellt werden sollte, wird mit einem Überprüfungsschritt 116 fortgefahren. In Schritt 116 wird ermittelt, ob ein erster Zeitgeber (Timer), der beispielsweise bei einem vorhergehenden Betriebsvorgang oder im Initialisierungsschritt 104 gestartet wurde, abgelaufen ist. Wenn in Schritt 116 festgestellt wird, dass der erste Timer abgelaufen ist, wird zu Schritt 118 verzweigt und ein im Gehäuseinneren angeordneter Temperatursensor ausgelesen. Der Temperatursensor ist auf der Leiterplatte der Motoransteuerschaltung nahe der temperaturempfindlichen Elektronikkomponenten angeordnet. Anschließend wird der ausgelesene Temperaturwert im Überprüfungsschritt 120 mit einer Temperaturobergrenze Tₘₐₓ von beispielsweise 80° verglichen. Allgemein kann die Temperaturobergrenze in einem Bereich zwischen 60° und 100° liegen.

Falls der Temperaturwert oberhalb der Temperaturobergrenze liegen sollte, wird zur Verhinderung eines Defekts oder einer Zerstörung von Elektronikkomponenten in Schritt 122 der Betrieb des Elektrowerkzeugs 10 vorübergehend gesperrt. Gleichzeitig kann mit einem akustischen Signal (welches sich von dem akustischen Signal von Schritt 114 unterscheidet) auf die vorübergehende Sperrung des Betriebs hingewiesen werden. Dann wird in den Schritten 124 und 126 die Temperatur wiederholt ausgelesen und mit der Temperaturobergrenze Tₘₐₓ verglichen. Die beiden Schritte 124 und 126 werden solange durchgeführt, bis die Temperaturobergrenze nicht mehr überschritten ist. Sobald dieser Fall eintritt, zweigt das Verfahren von Schritt 126 zurück zu Schritt 102.

Wird in Schritt 120 andererseits festgestellt, dass die Temperaturobergrenze nicht überschritten ist, oder ist der erste Zeitgeber (Schritt 116) noch nicht abgelaufen, wird das Betriebsverfahren mit einem Schritt 128 fortgesetzt, in dem ein zweiter Zeitgeber ausgelesen wird. Der zweite Zeitgeber, welcher ebenfalls beispielsweise mit dem letzten Betriebsvorgang oder im Initialisierungsschritt 104 gestartet wurde, gibt die Gültigkeitszeitdauer für eine frühere Sensorkalibrierung vor. Wird ein Ablauf dieser Zeitdauer ermittelt, so wird in Schritt 130 eine erneute Kalibrierung der Kraftsensoren durchgeführt. Die erneute Kalibrierung in Schritt 130 kann die in Schritt 110 ausgelesene Kraftwerte berücksichtigen und eine auf diesen Kraftwerten basierende Anpassung der entsprechenden Obergrenzen für die Überprüfung in Schritt 112 beinhalten. Wird in Schritt 128 ermittelt, dass keine erneute Kalibrierung erforderlich ist, oder wurde eine erneute Kalibrierung in Schritt 130 durchgeführt, wird das Betriebsverfahren mit Schritt 132 fortgesetzt.

In Schritt 132 werden die Kraftsensoren der beiden Betätigungsbaugruppen 14, 14' erneut ausgelesen. Wie bereits oben erläutert, steuert eine erste Betätigungsbaugruppe 14 die Ansteuerung des Elektromotors 22 in einer ersten Drehrichtung ("FWD"), während die zweite Betätigungsbaugruppe 14' den Betrieb des Elektromotors 22 in der entgegengesetzte Drehrichtung ("REV") steuert.

Nach dem Auslesen der Sensorwerte in Schritt 132 wird in Schritt 134 überprüft, ob der Kraftsensor der Betätigungsbaugruppe 14 ein Betätigungssignal ("FWD") ausgibt. Sollte dies der Fall sein, wird in einem sich daran anschließenden Schritt 136 ermittelt, ob der Kraftsensor der anderen Betätigungsbaugruppe 14' ebenfalls ein Betätigungssignal ("REV") ausgibt. Sollte in den Schritten 134 und 136 festgestellt werden, dass die Kraftsensoren beider Betätigungsbaugruppen 14, 14' Betätigungssignale ausgeben, wird wie oben erläutert darauf geschlossen, dass ein undefinierter Betätigungszustand vorliegt, da beide Betätigungseinheiten 14, 14' betätigt werden. Daraufhin erfolgt in Schritt 138 ein Abbremsen des Elektromotors 22 wie oben im Zusammenhang mit der Logikschaltung diskutiert. Sollte der Elektromotor 22 noch gar nicht laufen, verharrt der Elektromotor 22 in diesem Zustand. Im Anschluss an Schritt 138 werden in Schritt 140 die beiden Zeitgeber für die Temperaturüberprüfung und die Überprüfung einer erforderlichen erneuten Kalibrierung zurückgesetzt. Anschließen verzweigt das Betriebsverfahren zurück zu Schritt 116.

Wird in Schritt 134 festgestellt, dass der Kraftsensor der Betätigungsbaugruppe 14 nicht gedrückt ist und kann darüber hinaus in Schritt 142 ermittelt werden, dass auch der Kraftsensor der weiteren Betätigungsbaugruppe 14' nicht gedrückt ist, erfolgt in Schritt 144 ein Abbremsen des Elektromotors 22 analog zu Schritt 138, und das Verfahren fährt fort mit dem Überprüfungsschritt 116. Sollte hingegen in den Überprüfungsschritten 136, 142 ermittelt werden, dass lediglich einer der beiden Kraftsensoren der Betätigungsbaugruppen 14, 14' ein Betätigungssignal ausgibt, wird in Schritt 146 überprüft, ob ein Betriebsmodus gewählt wurde, in welchem eine Plausibilisierung der Kraftsensorsignale mittels des Schaltzustands der Schalter 46, 46' selektiv deaktiviert wurde. Im Fall einer deaktivierten Plausibilisierung verzweigt das Betriebsverfahren von Schritt 146 zu Schritt 148, und der Elektromotor 22 wird in der angeforderten Drehrichtung ("FWD"/"REV") gestartet. Anschließend erfolgt eine Motordrehzahlregelung in Abhängigkeit des von der entsprechenden Betätigungsbaugruppe 14, 14' ausgegebenen Signals (also betätigungskraftabhängig).

Wird andererseits in Schritt 146 festgestellt, dass ein Betriebsmodus aktiviert ist, in welchem eine Plausibilisierung durch Auswerten des Schaltzustands des entsprechenden Schalters 46, 46' erfolgt, wird in den Schritten 150 und 152 ermittelt, ob der Schalter 46, 46', welcher der das Betätigungssignal ausgebenden Betätigungsbaugruppe 14, 14' zugeordnet ist, sich in seinem geschlossenen Schaltzustand befindet. Sollte dies nicht der Fall sein, lässt dies auf eine Fehlfunktion schließen, da es nicht plausibel ist, dass bei offenem Schalter 46, 46' der Kraftsensor der zugeordneten Betätigungsbaugruppe 14, 14' ein Betätigungssignal liefert. Aus diesem Grund verzweigt das Verfahren in diesem Fall von Schritt 152 zu Schritt 138 und der Elektromotor 22 wird abgebremst oder gar nicht erst gestartet.

Wird andererseits im Rahmen der Plausibilitätskontrolle in Schritt 152 ermittelt, dass der Schalter 46, 46', welcher der das Betätigungssignal ausgebenden Betätigungsbaugruppe 14, 14' zugeordnet ist, sich in seinem geschlossenen Zustand befindet, ist die Plausibilitätsprüfung erfolgreich abgeschlossen und der Elektromotor wird in Schritt 148 in der angeforderten Drehrichtung gestartet. Außerdem wird dessen Drehzahl betätigungskraftabhängig geregelt.

Das beschriebene chirurgische Elektrowerkzeug 10 bietet aufgrund der zusätzlich zu den Kraftsensoren vorgesehenen Schalter 46, 46' eine erhöhte Funktionssicherheit, da unplausible Betriebszustände sicher erkannt werden können. Solche unplausiblen Betriebszustände können beispielsweise mit einer auf Stöße oder Schläge zurückzuführenden plastischen Deformation der Sensorkapseln 30, 30' einhergehen. Bei einer plastischen Deformation kann der zugehörige Kraftsensor nämlich ein Signal liefern, dass irrtümlich als Betätigungssignal interpretiert werden könnte. Allerdings ist die Auswertung des Schaltzustands der Schalter 46, 46' nicht auf die oben erläuterte Plausibilitätskontrolle beschränkt.

Um eine plastische Deformation der Sensorkapseln 30, 30' soweit wie möglich zu vermeiden, ist jeder Sensorkapsel 30, 30' ein mechanisches Dämpfungsglied 48A, 48A' in Krafteinleitungsrichtung vorgelagert. In dem hier beschriebenen Ausführungsbeispiel sind die Dämpfungsglieder 48A, 48A' als konvexe Dickenerhöhungen in die Abdeckung 48 integriert und definieren so einen haptisch gut erfassbaren Krafteinleitungsbereich. In anderen Ausführungsformen wäre es denkbar, die Dämpfungsglieder unterhalb der Abdeckung 48 vorzusehen (z.B. zwischen der Abdeckung 48 und jeder Sensorkapsel 30, 30').

Weitere Vorteile des hier beschriebenen Elektrowerkzeugs 10 bestehen in der verbesserten Abdichtung des Gehäuseinneren bezüglich Sterilisationsmedien. Diese verbesserte Abdichtung ist beispielsweise auf das Vorsehen zusätzlicher Dichtungselemente wie der Ringdichtungen 42, 42' sowie auf die abdichtende Funktion der Abdeckung 48 und der Trägerplatte 32 zurückzuführen. Weitere Vorteile ergeben sich aus der insgesamt erhöhten Stabilität der Betätigungsbaugruppen 14, 14', die unter Anderem auf die Verwendung der Trägerplatte 32 und des Lagerbauteils 34 zurückzuführen ist. Für den Fachmann ist offensichtlich, dass diese verschiedene Funktionalitäten und Vorteile unabhängig voneinander realisierbar sind. So lassen sich beispielsweise die verbesserten Abdichtfunktionen und die erhöhte Stabilität unabhängig von der Verwendung der Schalter 46, 46' realisieren.

Selbstverständlich ist der Einsatzbereich der hier vorgestellten Betätigungsbaugruppe nicht auf ein chirurgisches Elektrowerkzeug in Gestalt eines Schraubendrehers beschränkt. Vielmehr kann eine Betätigungsbaugruppe auch in anderen chirurgischen Elektrowerkzeugen wie Bohrern, Sägen, usw. Verwendung finden.

Es sind daher zahlreiche Modifikationen und Ergänzungen in Bezug auf die erfindungsgemäße Betätigungsbaugruppe und das erfindungsgemäße chirurgische Elektrowerkzeug möglich. Der Umfang der Erfindung ist ausschließlich durch den Schutzbereich der nachfolgenden Ansprüche beschränkt.

## Patentansprüche

1. Betätigungsbaugruppe (14) zur betätigungskraftabhängigen Steuerung des Betriebs eines chirurgischen Elektrowerkzeugs (10), umfassend
- einen zur Erfassung der Betätigungskraft ausgebildeten Kraftsensor (60);
- ein Trägerbauteil (54) für den Kraftsensor (60), das dem Kraftsensor (60) in einer Krafteinleitungsrichtung vorgelagert und in Kraft übertragender Weise mit diesem gekoppelt ist; **gekennzeichnet durch**
- ein mechanisches Dämpfungsglied (48A), das dem Trägerbauteil (54) in der Krafteinleitungsrichtung vorgelagert ist.

2. Betätigungsbaugruppe nach Anspruch 1, ferner umfassend eine Abdeckung (48), welche über dem Trägerbauteil (54) angeordnet ist, wobei das Dämpfungsglied (48A) in die Abdeckung (48) integriert oder zwischen der Abdeckung (48) und dem Trägerbauteil (54) angeordnet ist.

3. Betätigungsbaugruppe nach Anspruch 2, **dadurch gekennzeichnet, dass** sich die Abdeckung (48) im Wesentlichen senkrecht zur Krafteinleitungsrichtung und/oder im Wesentlichen parallel zum Trägerbauteil (54) erstreckt.

4. Betätigungsbaugruppe nach einem der vorhergehenden Ansprüche, **dadurch gekennzeichnet, dass** das Dämpfungsglied (48A) eine stellenweise Dickenerhöhung aufweist.

5. Betätigungsbaugruppe nach Anspruch 4, **dadurch gekennzeichnet, dass** die stellenweise Dickenerhöhung einen haptisch erfassbaren Einleitungsbereich für die Betätigungskraft definiert.

6. Betätigungsbaugruppe nach einem der vorhergehenden Ansprüche, **dadurch gekennzeichnet, dass** das Dämpfungsglied (48A) eine minimale Dicke von ungefähr 1,5 mm besitzt.

7. Betätigungsbaugruppe nach einem der vorhergehenden Ansprüche, **dadurch gekennzeichnet, dass** das Dämpfungsglied (48A) und/oder die optionale Abdeckung (48) gegenüber Sterilisationsmedien undurchlässig ist.

8. Betätigungsbaugruppe nach einem der vorhergehenden Ansprüche, **dadurch gekennzeichnet, dass** das Dämpfungsglied (48A) und/oder die optionale Abdeckung (48) ein elastisches Polymermaterial enthält.

9. Betätigungsbaugruppe nach einem der vorhergehenden Ansprüche, **dadurch gekennzeichnet, dass** das Trägerbauteil (54) von einem Abschnitt einer den Kraftsensor (60) zumindest bereichsweise umgebenden Sensorkapsel (30) gebildet ist.

10. Betätigungsbaugruppe nach einem der vorhergehenden Ansprüche, **dadurch gekennzeichnet, dass** die Betätigungsbaugruppe (14) eine eigenständig handhabbare Unterbaugruppe des Elektrowerkzeugs (10) bildet.

11. Chirurgisches Elektrowerkzeug (10), umfassend
- ein Gehäuse (12); und
- eine im Bereich des Gehäuses (12) angeordnete Betätigungsbaugruppe (14) nach einem der Ansprüche 1 bis 10.

12. Chirurgisches Elektrowerkzeug nach Anspruch 11 in Verbindung mit zumindest Anspruch 2, **dadurch gekennzeichnet, dass** das Gehäuse eine Öffnung (16A) zur Krafteinleitung in Richtung auf den Kraftsensor (60) besitzt, wobei die Abdeckung (48) die Öffnung (16A) gegenüber dem Eintritt von Sterilisationsmedien dicht verschließt.

13. Chirurgisches Elektrowerkzeug nach Anspruch 12, **dadurch gekennzeichnet, dass** die Abdeckung (48) eine die Öffnung (16A) des Gehäuses (12) umgebende erste Oberflächenprofilierung aufweist, die im Wesentlichen formschlüssig mit einer zweiten Oberflächenprofilierung des Gehäuses (12) zusammenwirkt.

14. Chirurgisches Elektrowerkzeug nach Anspruch 12 oder 13, **dadurch**
**gekennzeichnet, dass** die Abdeckung (48) dichtend zwischen einer ins Gehäuseinnere weisenden ersten Oberfläche des Gehäuses (12) und einer der
ersten Oberfläche zugewandten zweiten Oberfläche der Betätigungsbaugruppe (14) angeordnet ist.

15. Chirurgisches Elektrowerkzeug nach Anspruch 14, **dadurch gekennzeichnet, dass** die erste Oberfläche an einer Auskragung (16) des Gehäuses (12) und/oder die zweite Oberfläche an einem Lagerglied (34) für das Trägerbauteil (54) ausgebildet ist.

## Claims

1. An actuation assembly (14) for the actuation-force-dependent control of the operation of a surgical power tool (10), comprising
- a force sensor (60) configured to sense the actuation force;
- a carrier component (54) for the force sensor (60) which is arranged upstream of the force sensor (60) in a direction of force application and is coupled thereto in a force-transmitting manner;
**characterised by**
- a mechanical damping member (48A) which is arranged upstream of the carrier component (54) in the direction of force application.

2. The actuation assembly according to claim 1, further comprising a cover (48) which is arranged above the carrier component (54), the damping member (48A) being integrated into the cover (48) or arranged between the cover (48) and the carrier component (54).

3. The actuation assembly according to claim 2, **characterised in that** the cover (48) extends substantially perpendicularly to the direction of force application and/or substantially parallel to the carrier component (54).

4. The actuation assembly according to one of the preceding claims, **characterised in that** the damping member (48A) has an increase in thickness in places.

5. The actuation assembly according to claim 4, **characterised in that** the increase in thickness in places defines a haptically detectable application region for the actuation force.

6. The actuation assembly according to one of the preceding claims, **characterised in that** the damping member (48A) has a minimum thickness of approximately 1.5 mm.

7. The actuation assembly according to one of the preceding claims, **characterised in that** the damping member (48A) and/or the optional cover (48) is impervious to sterilisation media.

8. The actuation assembly according to one of the preceding claims, **characterised in that** the damping member (48A) and/or the optional cover (48) contains an elastic polymer material.

9. The actuation assembly according to one of the preceding claims, **characterised in that** the carrier component (54) is formed by a section of a sensor capsule (30), which capsule surrounds the force sensor (60) at least in regions.

10. The actuation assembly according to one of the preceding claims, **characterised in that** the actuation assembly (14) forms an independently handleable subassembly of the power tool (10).

11. A surgical power tool (10), comprising
- a housing (12); and
- an actuation assembly (14) according to one of claims 1 to 10 which is arranged in the region of the housing (12).

12. The surgical power tool according to claim 11 in conjunction with at least claim 2, **characterised in that** the housing has an opening (16A) for the application of force in the direction of the force sensor (60), the cover (48) closing the opening (16A) in a manner sealed against the ingress of sterilisation media.

13. The surgical power tool according to claim 12, **characterised in that** the cover (48) has a first surface profiling which surrounds the opening (16A) of the housing (12) and which cooperates in a substantially form-fitting manner with a second surface profiling of the housing (12).

14. The surgical power tool according to claim 12 or 13, **characterised in that** the cover (48) is arranged sealingly between a first surface of the housing (12) facing into the housing interior and a second surface of the actuation assembly (14) facing the first surface.

15. The surgical power tool according to claim 14, **characterised in that** the first surface is formed on a projection (16) of the housing (12) and/or the second surface is formed on a bearing member (34) for the carrier component (54).

## Revendications

1. Ensemble d'actionnement (14) servant à commander le fonctionnement d'un outil électrochirurgical (10) en fonction de la force d'actionnement, comprenant
- un capteur de force (60) conçu pour détecter la force d'actionnement,
- une pièce support (54) pour ledit capteur de force (60), laquelle pièce est montée en amont du capteur de force (60) dans un sens d'introduction de la force et couplée avec celui-ci en transmission de force, **caractérisée par**
- un organe amortisseur mécanique (48A) monté en amont de ladite pièce support (54) dans le sens d'introduction de la force.

2. Ensemble d'actionnement selon la revendication 1, comprenant en outre un cache (48) disposé au-dessus de la pièce support (54), ledit organe amortisseur (48A) étant intégré dans le cache (48) ou disposé entre le cache (48) et la pièce support (54).

3. Ensemble d'actionnement selon la revendication 2, **caractérisée en ce que** ledit cache (48) s'étend pour l'essentiel perpendiculairement au sens d'introduction de la force et/ou pour l'essentiel parallèlement à la pièce support (54).

4. Ensemble d'actionnement selon l'une des revendications précédentes, **caractérisé en ce que** l'organe amortisseur (48A) présente par endroits une épaisseur accrue.

5. Ensemble d'actionnement selon la revendication 4, **caractérisé en ce que** ladite épaisseur accrue par endroits définit une zone d'introduction haptiquement détectable pour la force d'actionnement.

6. Ensemble d'actionnement selon l'une des revendications précédentes, **caractérisé en ce que** l'organe amortisseur (48A) possède une épaisseur minimale d'environ 1,5 mm.

7. Ensemble d'actionnement selon l'une des revendications précédentes, **caractérisé en ce que** l'organe amortisseur (48A) et/ou le cache optionnel (48) sont imperméables aux fluides de stérilisation.

8. Ensemble d'actionnement selon l'une des revendications précédentes, **caractérisé en ce que** l'organe amortisseur (48A) et/ou le cache optionnel (48) contiennent une matière polymère élastique.

9. Ensemble d'actionnement selon l'une des revendications précédentes, **caractérisé en ce que** la pièce support (54) est constituée par un segment d'une capsule de capteur (30) entourant au moins partiellement le capteur de force (60).

10. Ensemble d'actionnement selon l'une des revendications précédentes, **caractérisé en ce que** l'ensemble d'actionnement (14) forme un sous-ensemble de l'outil électrique (10), lequel sous-ensemble peut être manipulé de manière indépendante.

11. Outil électrochirurgical (10), comprenant
- un boîtier (12) et
- un ensemble d'actionnement (14) selon l'une des revendications 1 à 10, situé dans la zone dudit boîtier (12).

12. Outil électrochirurgical selon la revendication 11 en liaison avec au moins la revendication 2, **caractérisé en ce que** le boîtier possède une ouverture (16A) pour l'introduction de la force en direction du capteur de force (60), le cache (48) fermant ladite ouverture (16A) de manière étanche pour empêcher la pénétration de fluides de stérilisation.

13. Outil électrochirurgical selon la revendication 12, **caractérisé en ce que** le cache (48) présente un premier profilage superficiel entourant l'ouverture (16A) du boîtier (12), lequel profilage coopère pour l'essentiel par complémentarité de forme avec un deuxième profilage superficiel du boîtier (12).

14. Outil électrochirurgical selon la revendication 12 ou 13, **caractérisé en ce que** le cache (48) est disposé de manière étanche entre une première surface du boîtier (12) montrant vers l'intérieur de ce dernier et une deuxième surface de l'ensemble d'actionnement (14) tournée ver ladite première surface.

15. Outil électrochirurgical selon la revendication 14, **caractérisé en ce que** la première surface est réalisée sur une saillie (16) du boîtier (12), et/ou ladite deuxième surface sur un organe de palier (34) pour la pièce support (54).
